(19)

Europäisches
Patentamt
European
Patent Office
Office européen
des brevets

(11) **EP 4 183 322 A1**

(12) **EUROPEAN PATENT APPLICATION**
published in accordance with Art. 153(4) EPC

(43) Date of publication:
**24.05.2023 Bulletin 2023/21**

(21) Application number: **21857966.2**

(22) Date of filing: **08.03.2021**

(51) International Patent Classification (IPC):
**A61B 3/113** (2006.01)  **G06F 3/0346** (2013.01)
**G06F 3/038** (2013.01)

(52) Cooperative Patent Classification (CPC):
**A61B 3/113; G06F 3/0346; G06F 3/038**

(86) International application number:
**PCT/JP2021/008894**

(87) International publication number:
**WO 2022/038814 (24.02.2022 Gazette 2022/08)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.08.2020 JP 2020139346**

(71) Applicant: **JVCKenwood Corporation
Yokohama-shi, Kanagawa 2210022 (JP)**

(72) Inventor: **HAKOSHIMA, Shuji
Yokohama-shi, Kanagawa 221-0022 (JP)**

(74) Representative: **Schmidbauer, Andreas Konrad
Wagner & Geyer Partnerschaft mbB
Patent- und Rechtsanwälte
Gewürzmühlstrasse 5
80538 München (DE)**

(54) **CORNEAL CURVATURE RADIUS CALCULATION DEVICE, LINE-OF-SIGHT DETECTION DEVICE, CORNEAL CURVATURE RADIUS CALCULATION METHOD, AND CORNEAL CURVATURE RADIUS CALCULATION PROGRAM**

(57) A cornea curvature radius calculation device includes a first light source and a second light source that emit detection light from positions different from each other and that apply the detection light to at least one of eyeballs of a subject; an imaging unit that captures an image of the eyeball of the subject to which the detection light is applied; a position calculator that, based on the image of the eyeball of the subject that is captured by the imaging unit, calculates each of a position of a first cornea reflection center according to the detection light from the first light source and a position of a second cornea reflection center according to the detection light from the second light source; a center distance calculator that calculates a center-center distance between the position of the first cornea reflection center and the second cornea reflection center; and a cornea curvature radius calculator that, based on the center-center distance and a distance between the imaging unit and the eyeball of the subject, calculates a cornea curvature radius of the eyeball of the subject.

FIG.6

$$\theta1 = \frac{1}{2} \cdot arctan(a/x)$$
$$\theta2 = \frac{1}{2} \cdot arctan(b/x)$$
$$d = r \cdot sin\theta1 + r \cdot sin\theta2$$

EP 4 183 322 A1

**Description**

Field

The present disclosure relates to a cornea curvature radius calculation device, a line-of-sight detection device, a cornea curvature radius calculation method, and a cornea curvature radius calculation program. Background

A line-of-sight detection device that, by a plurality of light sources, emits detection light and applies the detection light to an eyeball of a subject, by an imaging unit, acquires an image of the eyeball to which the detection light is applied, and detects a direction of the line of sight of the subject based on the acquired image and the distance between the imaging unit and the subject has been known (for example, see Patent Literature 1) .

Citation List

Patent Literature

Patent Literature 1: Japanese Laid-open Patent Publication No. 2020-38734

Summary

Technical Problem

The line-of-sight detection device described above performs a calibration process of calculating a cornea curvature radius of the subject before performing a line-of-sight detection process of detecting a direction of the light of sight of the subject. In the calibration process, for example, it is necessary to display a target image on a display unit to cause the subject to gaze the target image. For this reason, a configuration that makes it possible to efficiently calculate a cornea curvature radius of a subject is required.

The disclosure was made in view of the above-described circumstances and an object of the disclosure is to provide a cornea curvature radius calculation device, a line-of-sight detection device, a cornea curvature radius calculation method, and a cornea curvature radius calculation program that make it possible to more efficiently calculate a cornea curvature radius of a subject.

Solution to Problem

A cornea curvature radius calculation device according to the present disclosure comprising: a first light source and a second light source that emit detection light from positions different from each other and that apply the detection light to at least one of eyeballs of a subject; an imaging unit that captures an image of the eyeball of the subject to which the detection light is applied; a position calculator that, based on the image of the eyeball of the subject that is captured by the imaging unit, calculates each of a position of a first cornea reflection center according to the detection light from the first light source and a position of a second cornea reflection center according to the detection light from the second light source; a center distance calculator that calculates a center-center distance between the position of the first cornea reflection center and the second cornea reflection center; and a cornea curvature radius calculator that, based on the center-center distance and a distance between the imaging unit and the eyeball of the subject, calculates a cornea curvature radius of the eyeball of the subject.

A line-of-sight detection device according to the present disclosure comprising: the cornea curvature radius calculation device above; and a detector processor that detects a position of a pupil center of the subject based on the image of the eyeball of the subject that is captured by the imaging unit of the cornea curvature radius calculation device and that detects a line of sight of the subject based on the detected position of the pupil center, the position of the first light source of the cornea curvature radius calculation device and the position of the second light source, the position of the first cornea reflection center and the position of the second cornea reflection center that are calculated by the position calculator of the cornea curvature radius calculation device, and the cornea curvature radius that is calculated by the cornea curvature radius calculator of the cornea curvature radius calculation device.

A cornea curvature radius calculation method according to the present disclosure comprising: emitting detection light from a first light source and a second light source that are arranged in positions different from each other and applying the detection light to at least one of eyeballs of a subject; by an imaging unit, capturing an image of the eyeball of the subject to which the detection light is applied; based on the captured image of the eyeball of the subject, calculating each of a position of a first cornea reflection center according to the detection light from the first light source and a position of a second cornea reflection center according to the detection light from the second light source; calculating a center-center distance between the position of the first cornea reflection center and the second cornea reflection center;

and based on the center-center distance and a distance between the imaging unit and the eyeball of the subject, calculating a cornea curvature radius of the eyeball of the subject.

A cornea curvature radius calculation program according to the present disclosure that causes a computer to execute a process of emitting detection light from a first light source and a second light source that are arranged in positions different from each other and applying the detection light to at least one of eyeballs of a subject; a process of, by a single imaging unit, capturing an image of the eyeball of the subject to which the detection light is applied; a process of, based on the captured image of the eyeball of the subject, calculating each of a position of a first cornea reflection center according to the detection light from the first light source and a position of a second cornea reflection center according to the detection light from the second light source; a process of calculating a center-center distance between the position of the first cornea reflection center and the second cornea reflection center; and a process of, based on the center-center distance and a distance between the imaging unit and the eyeball of the subject, calculating a cornea curvature radius of the eyeball of the subject.

Advantageous Effects of Invention

According to the disclosure, it is possible to calculate a cornea curvature radius of a subject more efficiently.

Brief Description of Drawings

FIG. 1 is a diagram schematically illustrating an example of a line-of-sight detection device according to an embodiment.
FIG. 2 is a functional block diagram illustrating an example of the line-of-sight detection device.
FIG. 3 is a diagram schematically illustrating a state in which detection light from a light source unit is applied to an eyeball of a subject.
FIG. 4 is a diagram schematically illustrating schematically illustrating a state in which detection light from the light source unit is applied to the eyeball of the subject.
FIG. 5 is a diagram schematically illustrating a state in which the detection light from the light source unit is applied to the eyeball of the subject.
FIG. 6 is a diagram illustrating an example of a positional relationship of the light source unit, an imaging unit, and the eyeball of the subject.
FIG. 7 is a diagram illustrating an example of image data on the eyeball that is captured by the imaging unit.
FIG. 8 is a schematic diagram for describing the principle of the line-of-sight detection process according to the embodiment.
FIG. 9 is a flowchart illustrating an example a cornea curvature radius calculation method according to the embodiment.
FIG. 10 is a flowchart illustrating an example the line-of-sight detection process in a line-of-sight detection method according to the embodiment.
FIG. 11 is a diagram illustrating an example of image data of eyeballs that is captured with a first light source and a second light source being turned on at the same timing.
FIG. 12 is a diagram illustrating an example of the image data of the eyeballs that is captured with the first light source and the second light source being turned on at the same timing.
FIG. 13 is a diagram illustrating an example of the image data of the eyeballs that is captured with the first light source and the second light source being turned on at the same timing.
FIG. 14 is a diagram illustrating an example of image data obtained by capturing an image of a state in which detection light is applied to an eyeball of a subject. Description of Embodiments

An embodiment of the disclosure will be described below based on the drawings. The embodiment does not limit the invention. The components in the embodiment described below include ones easily replaceable by those skilled in the art or ones substantially the same.
In the following description, description on a positional relationship among components will be given, setting a three-dimensional global coordinate system. A direction parallel to a first axis on a given plane is set for a X-axis direction, a direction parallel to a second axis on a given plane orthogonal to the first axis is set for a Y-axis direction, and a direction parallel to a third axis orthogonal to each of the first axis and the second axis is set for a Z-axis direction. The given planes include an X-Y plane.

Cornea Curvature Radius Calculation Device and Line-of-sight Detection Device

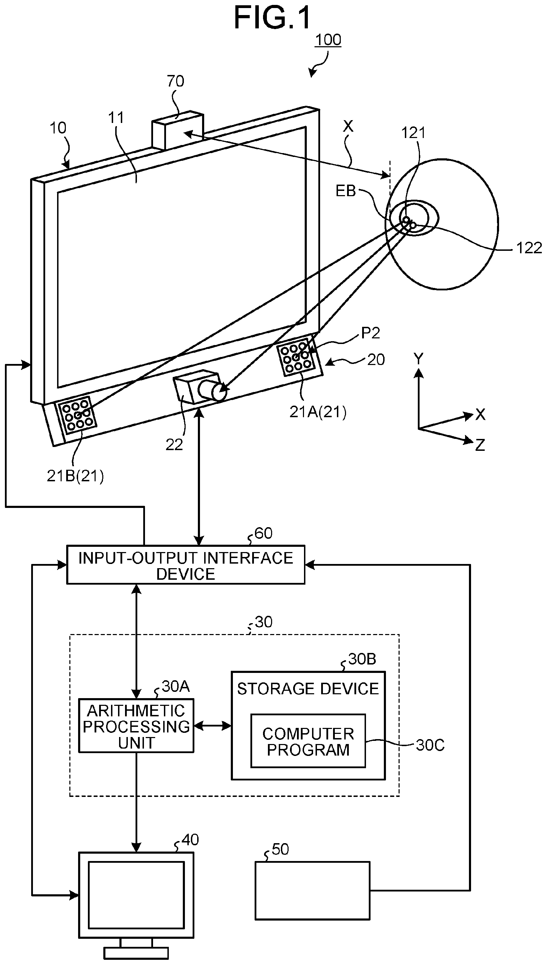

FIG. 1 is a diagram schematically illustrating an example of a line-of-sight detection device 100 according to the embodiment. The line-of-sight detection device 100 according to the embodiment detects lines of sight of a subject and outputs a detection result. The line-of-sight detection device 100 detects a line of sight, for example, based on a position of a pupil of the subject and a position of a cornea reflection image.

As illustrated in FIG. 1, the line-of-sight detection device 100 includes a display device 10, an image acquisition device 20, a computer system 30, an output device 40, an input device 50, an input-output interface device 60, and a camera-subject distance detector 70. The display device 10, the image acquisition device 20, the computer system 30, the output device 40, and the input device 50 perform data communications via the input-output interface device 60. Each of the display device 10 and the image acquisition device 20 includes a drive circuit not illustrated in the drawing. The camera-subject distance detector 70 detects the distance between an imaging unit 22 (to be described below) of the image acquisition device 20 and an eyeball of a subject. For example, an ultrasound sensor, a stereo camera, or the like, is used as the camera-subject distance detector 70; however, the camera-subject distance detector 70 is not limited to them and the camera-subject distance detector 70 may have another configuration as long as the distance between the imaging unit 22 and the eyeball of the subject can be detected. The site in which the camera-subject distance detector 70 is set may be a site other than one above the display device 10 and the camera-subject distance detector 70 may be set, for example, near the imaging unit 22. The camera-subject distance detector 70 need not be set.

The display device 10 includes a flat panel display, such as a liquid crystal display (LCD) or an organic electroluminescence display (OLED). In the embodiment, the display device 10 includes a display unit 11. The display unit 11 displays information, such as an image. The display unit 11 is substantially parallel to the X-Y plane. The X-axis direction is a left-right direction of the display unit 11, the Y-axis direction is an up-down direction of the display unit 11, and the Z-axis direction is a depth direction orthogonal to the display unit 11. The display device 10 may be a head mounted display device. In the case of a head-mounted display, such a configuration as that of the image acquisition device 20 is arranged in a head mounted module.

The image acquisition device 20 acquires image data on left and right eye balls EB of the subject and transmits the acquired image data to the computer system 30. The image acquisition device 20 includes a light source unit 21 and the imaging unit 22.

The light source unit 21 emits detection light to the eyeball EB of the subject. The light source unit 21 includes a first light source 21A and a second light source 21B that are arranged in positions different from each other. The first light source 21A and the second light source 21B contain LED (light emitting diode) light sources and are able to emit near-infrared light of, for example, a wavelength of 850 [nm]. The first light source 21A and the second light source 21B are able to control the timing of emission of the detection light.

The imaging unit 22 acquires image data by capturing images of the left and right eyeballs EB of the subject. In the embodiment, the single imaging unit 22 is provided. Various cameras corresponding to a method of detecting a line of sight of the subject are used as the imaging unit 22. As in the embodiment, in the case of a system that detects a line of sight based on a position of a pupil of a subject and a position of a corneal reflection image, the imaging unit 22 includes an infrared camera and includes an optical system that can transmit near-infrared light of a wavelength of, for example, 850 [nm] and an imaging device capable of receiving the near-infrared light. The imaging unit 22 outputs a frame synchronization signal. The period of the frame synchronization signal can be set at, for example, 20 [msec]; however, the period is not limited to this. In the embodiment, the imaging unit 22 is a single camera. The imaging unit 22 is arranged in a middle position between the first light source 21A and the second light source 21B. The image data that is acquired by the imaging unit 22 has a configuration in which pixels having luminances that are set by 8-bit values (0 to 255) are arrayed two-dimensionally. The luminance presents that, the smaller the value is, the darker it is and, the larger the value is, the brighter it is. A pixel whose value of luminance is 0 is displayed as black in the image data. A pixel whose value of luminance is 255 is displayed as white in the image data.

The computer system 30 has general control on operations of the line-of-sight detection device 100. The computer system 30 includes an arithmetic processing unit 30A and a storage device 30B. The arithmetic processing unit 30A includes a microprocessor, such as a CPU (central processing unit). The storage device 30B includes memories, such as a ROM (read only memory) and a RAM (random access memory), or a storage. The arithmetic processing unit 30A performs arithmetic processing according to a computer program 30C that is stored in the storage device 30B.

The output device 40 includes a display device, such as a flat panel display. Note that the output device 40 may include a printing device. The input device 50 generates input data by being operated. The input device 50 includes a keyboard or a mouse for computer systems. Note that the input device 50 may include a touch sensor with which the display unit of the output device 40 serving as a display device is provided.

The line-of-sight detection device 100 according to the embodiment is a device in which the display device 10 and the computer system 30 are independent from each other. Note that the display device 10 and the computer system 30 may be integrated. For example, the line-of-sight detection device 100 may include a tablet personal computer. In this case, a display device, an image acquisition device, a computer system, an input device, an output device, etc., may be installed in the tablet personal computer.

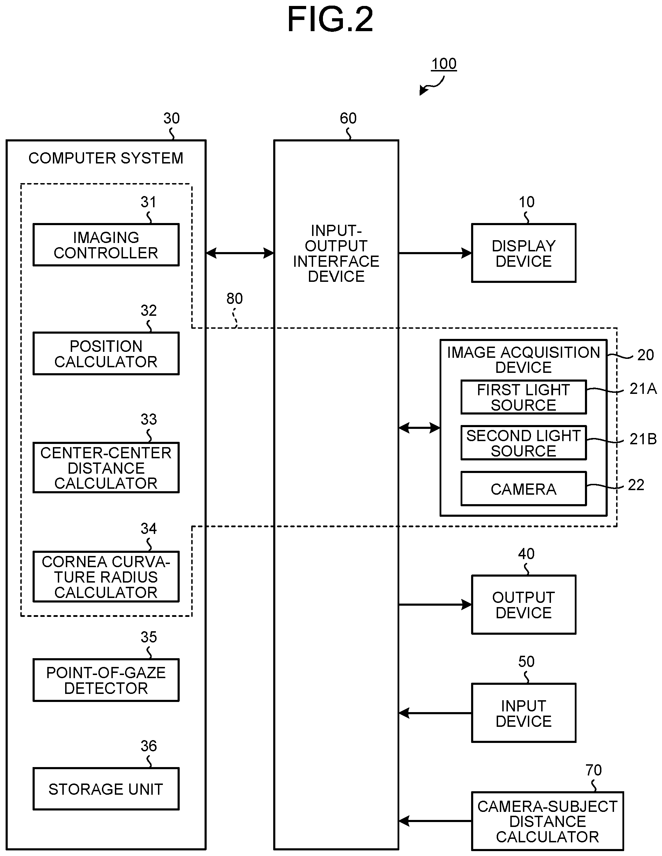

FIG. 2 is a functional block diagram illustrating an example of the line-of-sight detection device 100. As illustrated in FIG. 2, the computer system 30 includes an imaging controller 31, a position calculator 32, a center-center distance calculator 33, a cornea curvature radius calculator 34, a detection processor 35, and a storage unit 36. Functions of the computer system 30 are implemented by the arithmetic processing unit 30A and the storage device 30B (refer to FIG. 1). Part of the functions of the computer system 30 may be provided outside the line-of-sight detection device 100. In the embodiment, a cornea curvature radius calculation device 80 consists of the light source unit 21 (the first light source 21A and the second light source 21B) and the imaging unit 22 of the image acquisition device 20, the imaging controller 31, the position calculator 32, the center-center distance calculator 33, and the cornea curvature radius calculator 34. The cornea curvature radius calculation device 80 may include at least one of the detection processor 35 and the storage unit 36.

The imaging controller 31 controls the light source unit 21 and the imaging unit 22. The imaging controller 31 controls the timing of emission of the detection light, the time of emission, etc., with respect to each of the first light source 21A and the second light source 21B. The imaging controller 31 controls the timing of imaging, etc., with respect to the imaging unit 22. The imaging controller 31 causes the detection light to be emitted from the first light source 21A and the second light source 21B in synchronization with the period of the frame synchronization signal of the imaging unit 22. In the embodiment, the imaging controller 31 causes the first light source 21A and the second light source 21B to emit the detection light alternately, for example, in every period of the frame synchronization signal. The imaging controller 31 acquires image data that is acquired by the image acquisition device 20. The imaging controller 31 stores the acquired image data in the storage unit 36.

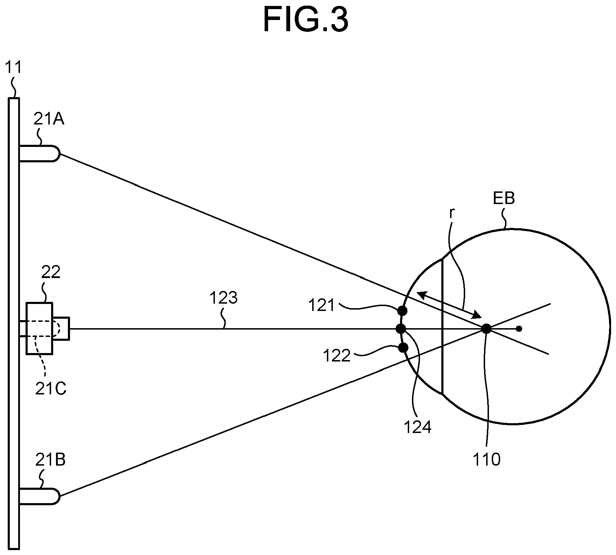

Based on the image data on the eyeballs of the subject that is captured by the imaging unit 22, the position calculator 32 calculates each of a position of a first cornea reflection center 121 (refer to FIG. 1 and FIG. 3) according to the detection light from the first light source 21A and a position of a second cornea reflection center 122 (refer to FIG. 1 and FIG. 3) according to the detection light from the second light source 21B. The first cornea reflection center 121 and the second cornea reflection center 122 are centers of cornea reflection images (a first cornea reflection image 120A and a second cornea reflection image 120B) according to the respective sets of the detection light. The position calculator 32 stores the calculated positions of the first cornea reflection center 121 and the second cornea reflection center 122 in the storage unit 36.

The center-center distance calculator 33 calculates a center-center distance between the position of the first cornea reflection center 121 and the second cornea reflection center 122.

Based on the center-center distance and the distance between the imaging unit 22 and the eyeball EB of the subject (referred to as a camera-subject distance below), the cornea curvature radius calculator 34 calculates a cornea curvature radius of the eyeball of the subject. The cornea curvature radius calculator 34 stores the calculated camera-subject distance in the storage unit 36. The cornea curvature radius is the distance between the surface of the cornea of the eyeball EB and a cornea curvature center.

The cornea curvature center is described here. In the embodiment, the case in which the eyeballs EB are illuminated with the first light source 21A and the second light source 21B and the single imaging unit 22 captures images of the eyeballs EB is described. Note that there is no limitation to the case with the single imaging unit and the same description applies also to the case with a single light source and two cameras.

FIG. 3 is a diagram schematically illustrating a state in which the detection light from the light source unit 21 is applied to the eyeballs EB of the subject. As illustrated in FIG. 3, in the embodiment, the first light source 21A and the second light source 21B of the light source unit 21 are arranged in positions that are symmetrical with respect to the imaging unit 22. Thus, in this case, it can be regarded that a virtual light source 21C is in the position of the imaging unit 22.

The first cornea reflection center 121 represents a corneal reflection center in an image in the case where the detection light is applied to the eyeball EB from the first light source 21A. The second cornea reflection center 122 represents a cornea reflection center in an image in the case where the detection light is applied to the eyeball EB from the second light source 21B. A cornea reflection center 124 represents a cornea reflection center corresponding to the virtual light source 21C.

The position of the cornea reflection center 124 is calculated based on the positions of the first cornea reflection center 121 and the second cornea reflection center 122. The detection processor 35 calculates a position of the imaging unit 22 on the image with respect to the first cornea reflection center 121 and the second cornea reflection center 122. Based on the camera-subject distance, the detection processor 35 transforms the position into a three-dimensional coordinate system. A transformation parameter for transforming a position into a three-dimensional coordinate system is stored in, for example, the storage unit 36. Based on the positions of the first cornea reflection center 121 and the second cornea reflection center 122 that are determined by the three-dimensional coordinate system, the detection processor 35 calculates a position of the cornea reflection center 124 in the three-dimensional coordinate system.

A cornea curvature center 110 is on a straight line 123 connecting the virtual light source 21C and the cornea reflection center 124. On the straight line 123, a position in which the distance from the cornea reflection center 124 is a given value serves as the position of the cornea curvature center 110. In the embodiment, the given value is a cornea curvature radius r. In other words, the cornea curvature radius r is the distance between the surface of the cornea and the cornea curvature center 110. The cornea curvature radius calculator 34 calculates a value of the cornea curvature radius r.

The detection processor 35 detects a position of a pupil center of the subject based on the image data that is captured by the imaging unit 22. The pupil center is the center of a pupil 112. The detection processor 35 detects a line-of-sight vector of the eyeball EB of the subject based on the positons of the first light source 21A and the second light source 21B, the positions of the first cornea reflection center 121 and the second cornea reflection center 122 that are calculated by the position calculator 32, and the value of the cornea curvature radius r that is calculated by the cornea curvature radius calculator 34.

Specifically, based on the positions of the first cornea reflection center 121 and the second cornea reflection center 122, the detection processor 35 calculates the position of the cornea reflection center 124 corresponding to the virtual light source 21C. The detection processor 35 finds the straight line 123 connecting the virtual light source 21C and the cornea reflection center 124 and finds, as a position of the cornea curvature center 110, a position on the straight line in which the distance from the cornea reflection center 124 is the value of the cornea curvature radius r. The detection processor 35 detects the straight line passing through the cornea curvature center 110 and the pupil center as the line of sight of the subject (line-of-sight vector). After detecting the line-of-sight vector, the detection processor 35 detects a position of a point of gaze representing the intersection between the line-of-sight vector and the display unit 11.

The storage unit 36 stores various types of data and programs for performing processing in each unit of the computer system 30. The storage unit 36 stores, for example, data on an image to be displayed on the display unit 11. The storage unit 36 stores each of the image data that is acquired by the imaging controller 31, the positions of the first cornea reflection center 121 and the second cornea reflection center 122 that are calculated by the position calculator 32, and the cornea curvature radius r that is calculated by the cornea curvature radius calculator 34.

The storage unit 36 also stores a cornea curvature radius calculation program that causes a computer to execute a process of emitting detection light from the first light source 21A and the second light source 21B that are arranged in the positions different from each other and applying the detection light to at least one of the eyeballs EB of a subject; a process of, by the single imaging unit 22, capturing an image of the eyeball EB of the subject to which the detection light is applied; a process of, based on the captured image of the eyeball of the subject, calculating each of a position of the first cornea reflection center 121 according to the detection light from the first light source 21A and a position of the second cornea reflection center 122 according to the detection light from the second light source 21B; a process of calculating a center-center distance between the positon of the first cornea reflection center 121 and the second cornea reflection center 122; and a process of, based on the center-center distance and a distance between the imaging unit 22 and the eyeball EB of the subject, calculating a cornea curvature radius r of the eyeball of the subject.

Cornea Curvature Radius Calculation Method

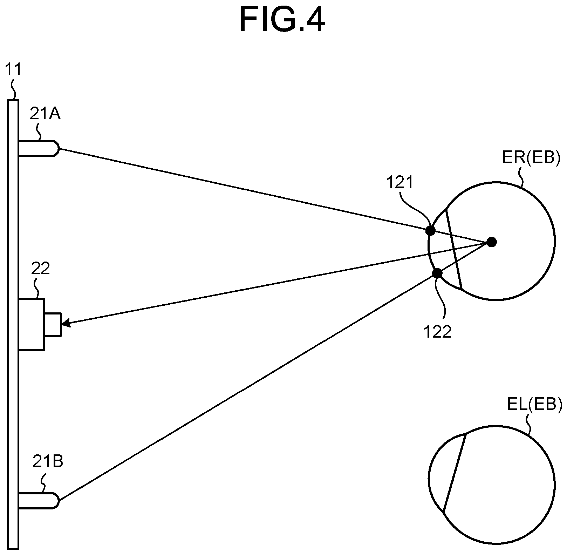

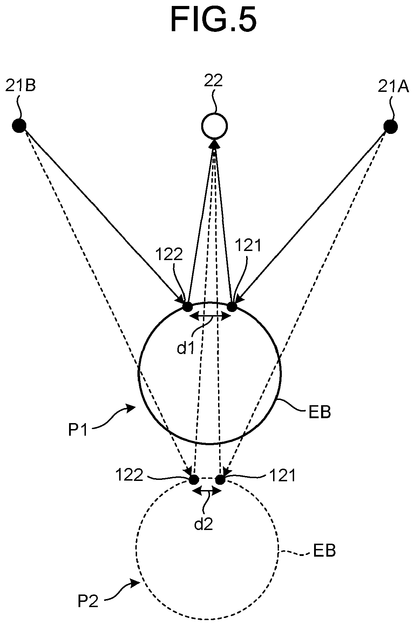

A cornea curvature radius calculation method according to the embodiment will be described next. FIG. 4 and FIG. 5 are diagrams schematically illustrating the state in which the detection light from the light source unit 21 is applied to the eyeball EB of the subject. The eyeball EB of the subject on the right (the right eyeball ER) will be described below and the eyeball EB on the left (the left eyeball EL) can be described similarly. As illustrated in FIG. 4 and FIG. 5, when the detection light is applied from the first light source 21A and the second light source 21B, a cornea reflection image is formed on the cornea of the eyeball EB. The center of the cornea reflection image of the first light source 21A is the first cornea reflection center 121. The center of the cornea reflection image of the second light source 21B is the second cornea reflection center 122.

As illustrated in FIG. 5, in association with variation in the distance between the imaging unit 22 and the subject, a center-center distance d between the first cornea reflection center 121 and the second cornea reflection center 122 varies. For example, d1>d2 holds when a camera-subject distance d1 in the case where the camera-subject distance that is the distance between the imaging unit 22 and the eyeball EB of the subject is x1 (a position P1) and a center-center distance d2 in the case where the camera-subject distance is x2 larger than x1 (a position P2).

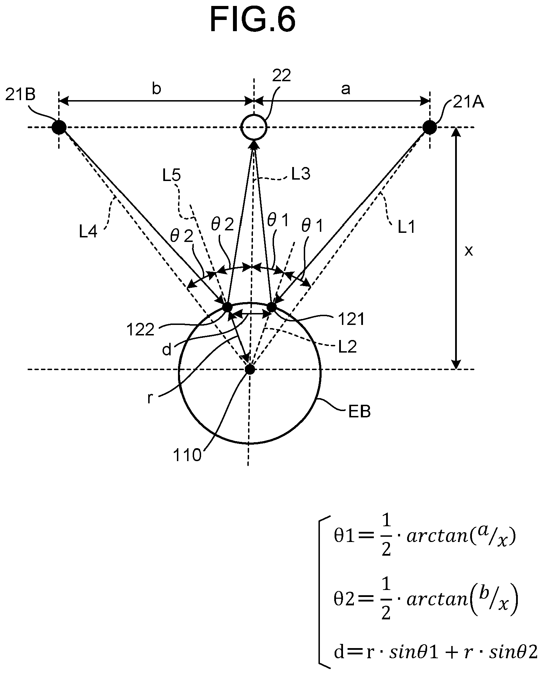

FIG. 6 is a diagram illustrating an example of a positional relationship of the light source unit, the imaging unit, and the eyeball of the subject. As illustrated in FIG. 6, a distance between the first light source 21A and the imaging unit 22 is referred to as a, a distance between the second light source 21B and the imaging unit 22 is referred to as b, a camera-subject distance between the imaging unit 22 and the subject is referred to as x, a distance between the cornea reflection center 121 and the cornea reflection center 122 that are detected in the eyeball EB is referred to as d, and a cornea curvature radius is referred to as r. In this example, the camera-subject distance x is a distance between the imaging unit 22 and the cornea curvature center 110 of the eyeball EB of the subject. Note that, for example, the value that is detected by the camera-subject distance detector 70 can be used as the camera-subject distance x. Here, because r<<x, it is possible to make an approximation of equality between an angle formed by a straight line L1 passing through the cornea curvature center 110 and the first light source 21A and a straight line L2 passing through the cornea curvature center 110 and the cornea reflection center 121 and an angle formed by a straight line L3 passing through the cornea curvature center 110 and the imaging unit 22 and the straight line 2. The angle is referred to as θ1 below. Similarly, it is possible to make an approximation of equality between an angle formed by a straight line L4 passing through the cornea curvature center 110 and the second light source 21B and a straight line L5 passing through the cornea curvature center 110 and the cornea reflection center 121 and an angle formed by the straight line L3 passing through the cornea curvature center 110 and the imaging unit 22 and the straight line 5. The angle is referred to as θ2 below.

In this case, Equation 1 holds from Equation 3.

$$\theta1=\arctan(a/x)/2 \qquad \cdots \text{(Equation 1)}$$

$$\theta2=\arctan(n/x)/2 \qquad \cdots \text{(Equation 2)}$$

$$d=r\cdot\sin\theta1+r\cdot\sin\theta2 \qquad \cdots \text{(Equation 3)}$$

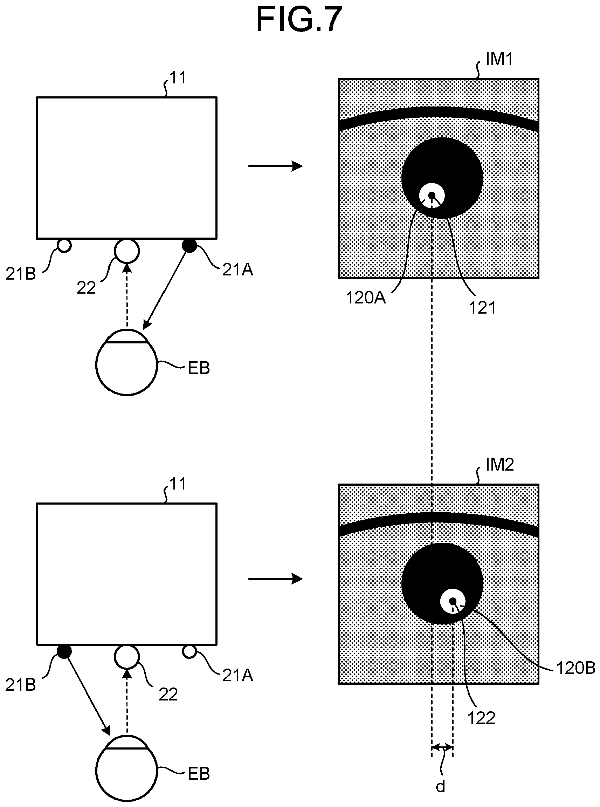

FIG. 7 is a diagram illustrating an example of the image data on the eyeball EB that is captured by the imaging unit 22. As illustrated in FIG. 7, in the embodiment, image data IM1 in the case where imaging is performed with the detection light from the first light source 21A being applied to the eyeball EB and image data IM2 in the case where imaging is performed with the detection light from the second light source 21B being applied to the eyeball EB are acquired as separate sets of image data. A cornea reflection image according to the detection light from the first light source 21A (referred to as the first cornea reflection image 120A below) appears in the image data IM1. A cornea reflection image according to the detection light from the second light source 21B (referred to as the second cornea reflection image 120B below) appears in the image data IM2. In the embodiment, the first light source 21A and the second light source 21B are turned on at different sets of timing. For this reason, as for appearance of the first cornea reflection image 120A and the second cornea reflection image 120B, only the first cornea reflection image 120A appears in the image data IM1 on one hand and only the second cornea reflection image 120B appears in the image data IM2 on the other hand.

Based on the acquired image data IM1, the position calculator 32 calculates a position of the first cornea reflection center 121 according to the detection light from the first light source 21A. Based on the acquired image data IM2, the position calculator 32 calculates a position of the second cornea reflection center 122 according to the detection light from the second light source 21B. In the sets of image data IM1 and IM2, the pixels from which the first cornea reflection image 120A and the second cornea reflection image 120B have higher luminances than those of other pixels. The position calculator 32 searches for a pixel with the highest luminance in the sets of image data IM1 and IM2, finds a high-luminance area where there are pixels whose luminances are within a given range based on the pixel, finds a luminance gravity center of the area, and sets sets of coordinates of the luminance gravity centers on the images as the first cornea reflection center 121 and the second cornea reflection center 122. Note that, it is schematically illustrated in FIG. 7 that the luminance of the high luminance area is being uniform; however, for example, the luminance may lower from one pixel in the high luminance area to surrounding pixels.

The center-center distance calculator 33 calculate an actual distance d (mm) between the first cornea reflection center 121 and the second cornea reflection center 122 from a distance (the number of pixels of the imaging device) d' between the first cornea reflection center 121 contained in the image data IM1 and the second cornea reflection center 122 contained in the image data IM2. Note that, in FIG. 7, black points are represented in FIG. 7 in order to easily determine the positions of the first cornea reflection center 121 and the second cornea reflection center 122; however, there is no black point actually.

The following Equation 4 holds when a focal length of a lens configuring the imaging unit 22 is f and a distance between pixels of the imaging device configuring the imaging unit 22 (pitch) is p.

$$d'=(f\cdot d/x)/p \qquad \cdots \text{(Equation 4)}$$

Solving Equation 4 about d' from Equation 1 described above leads to

$$d'=r \cdot (sin(arctan(a/x)/2)$$
$$+sin(arctan(b/x)/2)) \cdot f/(p \cdot x) \quad \cdots (Equation 5).$$

In Equation 5, for example, when a,b=100(mm), f=12(mm), x=600(mm), p=0.0048(mm/pixel), a relation between x and d' like Equation 6 below is found.

$$d'=r \cdot (2 \cdot sin(arctan(100/600)/2)) \cdot 12/(0.0048 \cdot 600)$$
$$=r \cdot \alpha \ (note \ that \ \alpha \ is \ a \ constant) \quad \cdots (Equation 6)$$

Using Equation 6 above makes it possible to calculate a cornea curvature radius r based on a center-center distance d'.

Line-of-sight Detection Method

An example of a line-of-sight detection method using the line-of-sight detection device 100 will be described next. In the line-of-sight detection method in the embodiment, as described above, a center-center distance between the first cornea reflection center 121 and the second cornea reflection center 122 is found and, based on the center-center distance, a cornea curvature radius r can be calculated. For this reason, a calibration process like a conventional line-of-sight detection method, that is, a calibration process that is performed in a way that a target image is displayed on the display unit 11 and a subject is caused to gaze the target image is unnecessary. Thus, the line-of-sight detection process is performed without performing the calibration process.

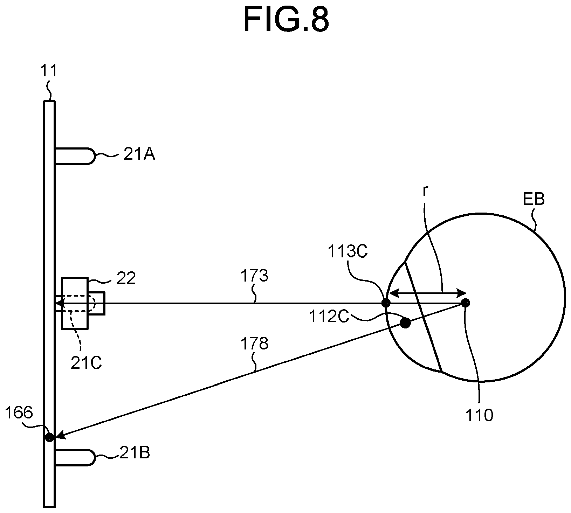

FIG. 8 is a schematic diagram for describing the principle of the line-of-sight detection process according to the embodiment. In the line-of-sight detection process, similarly to the calibration process, sets of detection light from the first light source 21A and the second light source 21B are emitted alternately and illuminate the eyeball EB and, by the imaging unit 22, an image of the eyeball EB is captured. Based on acquired image data on the eyeball EB, the detection processor 35 detects a position of a pupil center 112C and a positon of a cornea reflection center 113C. For example, using a camera-subject distance x that is detected by the camera-subject distance detector 70, etc., the detection processor 35 converts each of the positions of the pupil center 112C and the cornea reflection center 113C into a global coordinate system.

The detection processor 35 calculates a position of the cornea curvature center 110 based on a position of the virtual light source 21C, a position of the pupil center 112C, a position of the cornea reflection center 113C, and a cornea curvature radius r that is calculated in the calibration process. Specifically, the detection processor 35 finds a straight line 173 connecting the virtual light source 21C and the cornea reflection center 113C. The detection processor 35 finds, as a position of the cornea curvature center 110, a position separated from the cornea reflection center 113C with respect to an inner side of the eyeball EB by a distance corresponding to the cornea curvature radius r. The detection processor 35 finds a straight line 178 connecting the pupil center 112C and the cornea curvature center 110 and calculates a position of an intersection 166 of the straight line 178 and the display unit 11 as a position of a point of gaze.

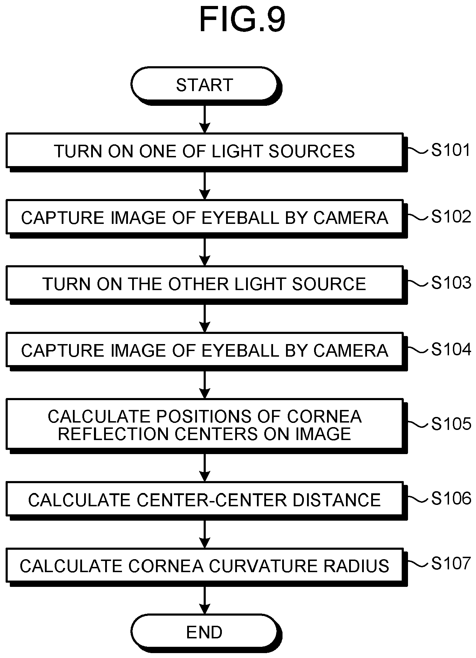

An example of the cornea curvature radius calculation method according to the embodiment will be described next with reference to FIG. 9. FIG. 9 is a flowchart illustrating an example a distance calculation method according to the embodiment. As illustrated in FIG. 9, according to control by the imaging controller 31, one of the first light source 21A and the second light source 21B is caused to emit light and detection light is applied to the eyeball EB (step S101) and, by the imaging unit 22, an image of the eyeball EB of the subject is captured (step S102). According to control by the imaging controller 31, the other one of the first light source 21A, the second light source 21B is caused to emit light and detection light is applied to the eyeball EB (step S103) and, by the imaging unit 22, an image of the eyeball EB of the subject is captured (step S104). The imaging controller 31 acquires image data on the left and right eyeballs EB that is captured by the imaging unit 22.

Based on the acquired image data, the position calculator 32 finds each of the first cornea reflection center 121 in the first cornea reflection image 120A and the second cornea reflection center 122 in the second cornea reflection image 120 with respect to the right and left eyeballs EB (step S105). The center-center distance calculator 33 calculates a center-center distance d' that is a distance between the first cornea reflection center 121 and the second cornea reflection center 122 (step S106). Based on the calculated center-center distance d' and a camera-subject distance x that is detected additionally, the cornea curvature radius calculator 34 calculates a cornea curvature radius r (step S107). As for the camera-subject distance x, for example, a value that is detected previously by the camera-subject distance detector 70, or the like, may be used as a constant or a camera-constant distance may be detected by the camera-subject distance detector 70, or the like, in every line-of-sight detection process.

In the application, the following description is left as an example of performing the line-of-sight detection process without calibration. Please have a look through it and, if you need to delete the description also in this application, we will delete it as instructed.

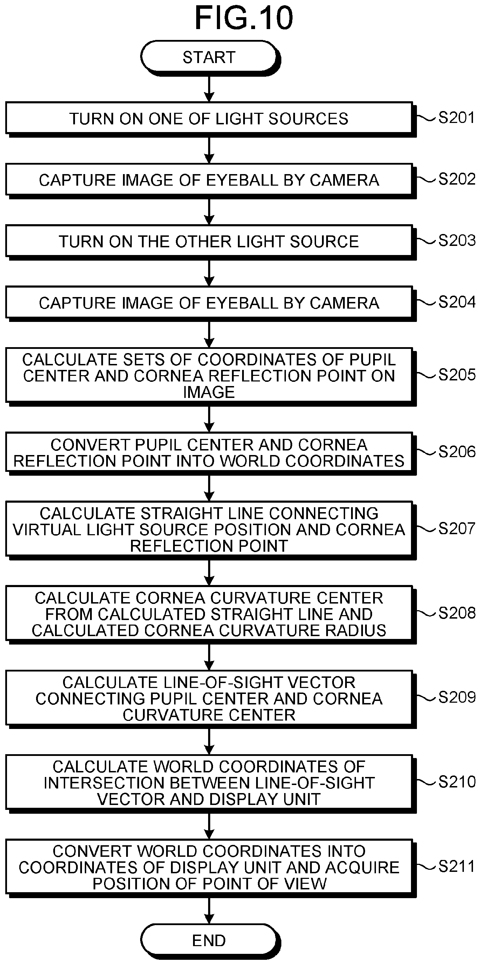

An example of the line-of-sight detection method according to the embodiment will be described next with reference to FIG. 10. FIG. 10 is a flowchart illustrating an example the line-of-sight detection process in the line-of-sight detection method according to the embodiment.

As illustrated in FIG. 10, in the line-of-sight detection process, according to control by the imaging controller 31, one of the first light source 21A and the second light source 21B is caused to emit light and detection light is applied to the eyeball EB (step S201) and, by the imaging unit 22, an image of the eyeball EB of the subject is captured (step S202). According to control by the imaging controller 31, the other one of the first light source 21A and the second light source 21B is caused to emit light, detection light is applied to the eyeball EB (step S203) and, by the imaging unit 22, an image of the eyeball EB of the subject is captured (step S204). The imaging controller 31 acquires image data on the left and right eyeballs EB that is captured by the imaging unit 22.

The detection processor 35 detects a position of the pupil center 112C and a position of the cornea reflection center 124 (step S205). Note that, as for the position of the cornea reflection center, positions (the first cornea reflection center 121 and the second cornea reflection center 122) that are detected by the position calculator 32 may be used. Using the camera-subject distance x described above, the detection processor 35 converts each of the positions into a global coordinate (world coordinate) system (step S206).

The detection processor 35 finds a straight line connecting the virtual light source 21C and the cornea reflection center 124 (step S207). The detection processor 35 calculates, as the cornea curvature center 110, a position that is on the back side of the eyeball EB by a value of a cornea curvature radius that is found by a calibration process from the position of the cornea reflection center 124 on the found straight line (step S208). The detection processor 35 calculates a straight line connecting the pupil center 112C and the cornea curvature center 110 as a line-of-sight vector (step S209) and calculates world coordinates of an intersection of the line-of-sight vector and the display unit 11 (step S210) . The detection processor 35 converts the calculated world coordinates into coordinates of the display unit 11, thereby acquiring a position of a point of gaze (step S211) .

As described above, the cornea curvature radius calculation device 80 according to the embodiment includes the first light source 21A and the second light source 21B that emit detection light from positions different from each other and apply the detection light to at least one of eyeballs EB of a subject; the imaging unit 22 that captures an image of the eyeball EB of the subject to which the detection light is applied; the position calculator 32 that, based on the image of the eyeball EB of the subject that is captured by the imaging unit 22, calculates each of a position of the first cornea reflection center 121 according to the detection light from the first light source 21A and a position of the second cornea reflection center 122 according to the detection light from the second light source 21B; the center-center distance calculator 33 that calculates a center-center distance d between the position of the first cornea reflection center 121 and the second cornea reflection center 122; and the cornea curvature radius calculator 34 that, based on the center-center distance d and a distance between the imaging unit 22 and the eyeball EB of the subject, calculates a cornea curvature radius r of the eyeball EB of the subject.

The cornea curvature radius calculation method according to the embodiment includes emitting detection light from the first light source 21A and the second light source 21B that are arranged in positions different from each other and applying the detection light to at least one of the eyeballs EB of a subject; by the imaging unit 22, capturing an image of the eyeball EB of the subject to which the detection light is applied; based on the captured image of the eyeball EB of the subject, calculating each of a position of the first cornea reflection center 121 according to the detection light from the first light source 21A and a position of the second cornea reflection center 122 according to the detection light from the second light source 21B; calculating a center-center distance d between the position of the first cornea reflection center 121 and the second cornea reflection center 122; and, based on the center-center distance d and a distance between the imaging unit 22 and the eyeball EB of the subject, calculating a cornea curvature radius r of the eyeball EB of the subject.

The cornea curvature radius calculation program according to the embodiment causes a computer to execute a process of emitting detection light from the first light source 21A and the second light source 21B that are arranged in positions different from each other and applying the detection light to at least one of the eyeballs EB of a subject; a process of, by the single imaging unit 22, capturing an image of the eyeball EB of the subject to which the detection light is applied; a process of, based on the captured image of the eyeball EB of the subject, calculating each of a position of the first cornea reflection center 121 according to the detection light from the first light source 21A and a position of the second cornea reflection center 122 according to the detection light from the second light source 21B; a process of calculating a center-center distance d' between the position of the first cornea reflection center 121 and the second cornea reflection center 122; and a process of, based on the center-center distance d' and a distance between the imaging unit 22 and the eyeball EB of the subject, calculating a cornea curvature radius r of the eyeball EB of the subject.

According to the above-described configuration, the center-center distance d' between the position of the first cornea reflection center 121 and the second cornea reflection center 122 is calculated based on the image data obtained by the imaging unit 22 and, based on the calculated center-center distance d' and the camera-subject distance x between the imaging unit 22 and the eyeball EB of the subject, the cornea curvature radius r of the eyeball EB of the subject is calculated and thus it is possible to more efficiently calculate the cornea curvature radius r of the subject without performing the calibration process, or the like.

In the cornea curvature radius calculation device 80 according to the embodiment, the first light source 21A and the second light source 21B emit the detection light alternately and the position calculator 32 calculates the position of the first cornea reflection center 121 based on the image that is captured at the timing at which the detection light is emitted from the first light source 21A and calculates the position of the second cornea reflection center 122 based on the image that is captured at the timing at which the detection light is emitted from the second light source 21B. When the detection light is emitted from the first light source 21A and the second light source 21B simultaneously, the first cornea reflection image 120A according to the detection light from the first light source 21A and the second cornea reflection image 120B according to the detection light from the second light source 21B overlap in some cases and there is a possibility that accuracy of detection of the first cornea reflection center 121 and the second cornea reflection center 122 would lower. On the other hand, the above-described configuration makes it possible to acquire the first cornea reflection image 120A and the second cornea reflection image 120B as different images and therefore it is possible to accurately detect the first cornea reflection center 121 and the second cornea reflection center 122.

The line-of-sight detection device 100 according to the embodiment includes the cornea curvature radius calculation device 80 and the detection processor 35 that detects the position of the pupil center of the subject based on the image of the eyeball EB of the subject that is captured by the imaging unit 22 and detects the line of sight of the subject based on the detected position of the pupil center, the position of the first light source 21A and the position of the second light source 21B, the position of the first cornea reflection center 121 and the position of the second cornea reflection center 122 that are calculated by the position calculator 32, and the cornea curvature radius r that is calculated by the cornea curvature radius calculator 34.

According to the above-described configuration, because it is possible to calculate the cornea curvature radius r of the subject using the cornea curvature radius calculation device 80, it is possible to perform the line-of-sight detection process of detecting a line of sight of a subject without performing the calibration process. In this manner, because calibration can be omitted as described above, it is possible to effectively detect the line of sight of the subject.

The technical scope of the disclosure is not limited to the above-described embodiment and changes can be added as appropriate without departing from the scope of the disclosure. For example, in the above-described embodiment, the case where the first light source 21A and the second light source 21B are caused to turn on is exemplified; however, embodiments are not limited to this and the imaging unit 22 may capture an image with the first light source 21A and the second light source 21B being caused to turn on at the same timing.

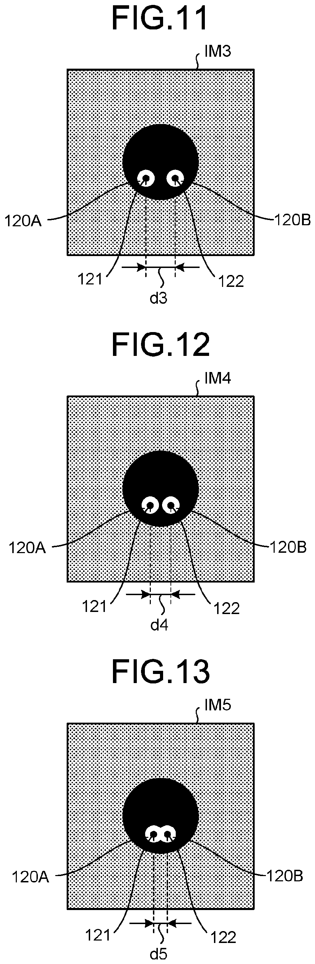

FIGS. 11 to 13 are diagrams illustrating examples of image data on the eyeball EB that is captured with the first light source 21A and the second light source 21B being turned on at the same timing. FIG. 11 illustrates an example of the case where, for example, the camera-subject distance between the imaging unit 22 and the subject is the same as that in FIG. 7. FIG. 12 illustrates an example of the case where the camera-subject distance is shorter than that in the state in FIG. 11. FIG. 13 illustrates an example of the case where the camera-subject distance is much shorter than that in the state in FIG. 12. In FIGS. 11 to 13, the first cornea reflection center 121 and the second cornea reflection center 122 are presented in black dots for easy determination of their positions; however, there are no black points actually.

In image data IM3 and IM4 illustrated in FIGS. 11 and 12, the image of the first cornea reflection image 120A and the image of the second cornea reflection image 120B are separated from each other. In this case, the position calculator 32 is able to find the image of the first cornea reflection image 120A and the image of the second cornea reflection image 120B as separate high-luminance areas and find sets of coordinates of luminance gravity centers of the separate high-luminance areas independently. Accordingly, it is possible to calculate positions of the first cornea reflection center 121 and the second cornea reflection center 122 separately from the single set of image data IM3 or IM4 and calculate center-center distances d3 or d4.

On the other hand, in image data IM5 illustrated in FIG. 13, the image of the first cornea reflection image 120A and the image of the second cornea reflection image 120B are joined partly. In this state, the position calculator 32 has difficulties in finding the image of the first cornea reflection image 120A and the image of the second cornea reflection image 120B as separate high-luminance areas. Accordingly, in such a case, using the image data IM1 and IM2 obtained by capturing the images of the eyeball EB with the first light source 21A and the second light source 21B being turned on at different sets of timing makes it possible to calculate the positions of the first cornea reflection center 121 and the second cornea reflection center 122 assuredly and calculate a center-center distance d5.

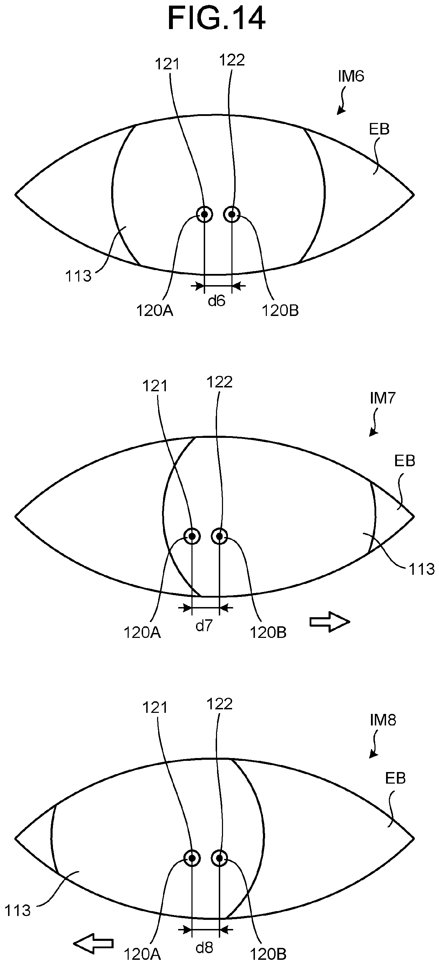

In the above-described embodiment, the case where a cornea curvature radius r is calculated when the line of sight of the subject is in one direction is exemplified and is described; however, embodiments are not limited to this. FIG. 14 is a diagram illustrating an example of image data obtained by capturing images of a state in which the detection light is applied to an eyeball of a subject.

In the example illustrated in image data IM6 in FIG. 14, when the subject looks ahead, for example, the first cornea reflection image 120A and the second cornea reflection image 120B are formed in a center part of a cornea 113 of the subject in the left-right direction. In this case, a center-center distance d6 (the distance between the first cornea reflection center 121 and the second cornea reflection center 122) in the center part of the cornea 113 of the subject is calculated.

In the example illustrated in image data IM7 in FIG. 14, when the subject looks left (a left direction to the subject: the right side in the drawing), for example, the first cornea reflection image 120A and the second cornea reflection image 120B are formed on a right-side part of the cornea 113 of the subject. In this case, a center-center distance d7 (the distance between the first cornea reflection center 121 and the second cornea reflection center 122) in the right-side part of the cornea 113 of the subject is calculated.

In the example illustrated in image data IM8 in FIG. 14, when the subject looks right (a right direction to the subject: the left side in the drawing), for example, the first cornea reflection image 120A and the second cornea reflection image 120B are formed on a left-side part of the cornea 113 of the subject. In this case, a center-center distance d8 (the distance between the first cornea reflection center 121 and the second cornea reflection center 122) in the left-side part of the cornea 113 of the subject is calculated.

The value of the cornea curvature radius r, for example, sometimes varies depending on the position of the eyeball EB (the position of the cornea 113). As described above, applying the detection light to the eyeball EB while varying the direction of the line of sight of the subject as described above makes it possible to find center-center distances d in different spots in the cornea 113 and furthermore a cornea curvature radius r. Accordingly, for example, in the line-of-sight detection process, it is possible to use the cornea curvature radius r corresponding to the position in the cornea 113 and thus obtain a more accurate detection result.

In the above-described embodiment, the configuration in which there is the single imaging unit 22 is exemplified and described; however, embodiments are not limited to this. A configuration in which two or more the imaging units 22 are provided may be employed.

Industrial Applicability

The cornea curvature radius calculation device, the line-of-sight detection device, the cornea curvature radius calculation method, and the cornea curvature radius calculation program according to the disclosure are usable for, for example, a processing device, such as a computer, or the like.

Reference Signs List

EB EYEBALL, d,d1,d2,d3,d4,d5,d6,d7,d8 CENTER-CENTER DISTANCE, IM1,IM2,IM3,IM4,IM5,IM6,IM7,IM8 IMAGE DATA, r CORNEA CURVATURE RADIUS, x CAMERA-SUBJECT DISTANCE, 10 DISPLAY DEVICE, 11 DISPLAY UNIT, 20 IMAGE ACQUISITION DEVICE, 21 LIGHT SOURCE UNIT, 21A FIRST LIGHT SOURCE, 21B SECOND LIGHT SOURCE, 21C VIRTUAL LIGHT SOURCE, 22 IMAGING UNIT, 30 COMPUTER SYSTEM, 30A ARITHMETIC PROCESSING UNIT, 30B STORAGE DEVICE, 30C COMPUTER PROGRAM, 31 IMAGING CONTROLLER, 32 POSITION CALCULATOR, 33 CENTER-CENTER DISTANCE CALCULATOR, 34 CORNEA CURVATURE RADIUS CALCULATOR, 35 DETECTION PROCESSOR, 36 STORAGE UNIT, 40 OUTPUT DEVICE, 50 INPUT DEVICE, 60 INPUT-OUTPUT INTERFACE DEVICE, 70 CAMERA-SUBJECT DISTANCE DETECTOR, 80 CORNEA CURVATURE RADIUS CALCULATION DEVICE, 100 LINE-OF-SIGHT DETECTION DEVICE, 110 CORNEA CURVATURE CENTER, 112 PUPIL, 112C PUPIL CENTER, 113 CORNEA, 113C,121,122,124 CORNEA REFLECTION CENTER, 120A FIRST CORNEA REFLECTION IMAGE, 120B SECOND CORNEA REFLECTION IMAGE, 121 FIRST CORNEA REFLECTION CENTER, 122 SECOND CORNEA REFLECTION CENTER

**Claims**

1.   A cornea curvature radius calculation device comprising:

a first light source and a second light source that emit detection light from positions different from each other and that apply the detection light to at least one of eyeballs of a subject;
an imaging unit that captures an image of the eyeball of the subject to which the detection light is applied;
a position calculator that, based on the image of the eyeball of the subject that is captured by the imaging unit, calculates each of a position of a first cornea reflection center according to the detection light from the first light source and a position of a second cornea reflection center according to the detection light from the second light source;

a center distance calculator that calculates a center-center distance between the position of the first cornea reflection center and the second cornea reflection center; and

a cornea curvature radius calculator that, based on the center-center distance and a distance between the imaging unit and the eyeball of the subject, calculates a cornea curvature radius of the eyeball of the subject.

2. The cornea curvature radius calculation device according to claim 1, wherein

the first light source and the second light source emit the detection light alternately,

the position calculator calculates the position of the first cornea reflection center based on an image that is captured at timing at which the detection light is emitted from the first light source and calculates the position of the second cornea reflection center based on an image that is captured at timing at which the detection light is emitted from the second light source.

3. A line-of-sight detection device comprising:

the cornea curvature radius calculation device according to claim 1 or 2; and

a detector processor that detects a position of a pupil center of the subject based on the image of the eyeball of the subject that is captured by the imaging unit of the cornea curvature radius calculation device and that detects a line of sight of the subject based on the detected position of the pupil center, the position of the first light source of the cornea curvature radius calculation device and the position of the second light source, the position of the first cornea reflection center and the position of the second cornea reflection center that are calculated by the position calculator of the cornea curvature radius calculation device, and the cornea curvature radius that is calculated by the cornea curvature radius calculator of the cornea curvature radius calculation device.

4. A cornea curvature radius calculation method comprising:

emitting detection light from a first light source and a second light source that are arranged in positions different from each other and applying the detection light to at least one of eyeballs of a subject;

by an imaging unit, capturing an image of the eyeball of the subject to which the detection light is applied;

based on the captured image of the eyeball of the subject, calculating each of a position of a first cornea reflection center according to the detection light from the first light source and a position of a second cornea reflection center according to the detection light from the second light source;

calculating a center-center distance between the position of the first cornea reflection center and the second cornea reflection center; and

based on the center-center distance and a distance between the imaging unit and the eyeball of the subject, calculating a cornea curvature radius of the eyeball of the subject.

5. A cornea curvature radius calculation program that causes a computer to execute

a process of emitting detection light from a first light source and a second light source that are arranged in positions different from each other and applying the detection light to at least one of eyeballs of a subject;

a process of, by a single imaging unit, capturing an image of the eyeball of the subject to which the detection light is applied;

a process of, based on the captured image of the eyeball of the subject, calculating each of a position of a first cornea reflection center according to the detection light from the first light source and a position of a second cornea reflection center according to the detection light from the second light source;

a process of calculating a center-center distance between the position of the first cornea reflection center and the second cornea reflection center; and

a process of, based on the center-center distance and a distance between the imaging unit and the eyeball of the subject, calculating a cornea curvature radius of the eyeball of the subject.

# FIG.1

# FIG.2

COMPUTER SYSTEM 30

IMAGING CONTROLLER 31

POSITION CALCULATOR 32

CENTER-CENTER DISTANCE CALCULATOR 33

CORNEA CURVA-TURE RADIUS CALCULATOR 34

POINT-OF-GAZE DETECTOR 35

STORAGE UNIT 36

INPUT-OUTPUT INTERFACE DEVICE 60

80

100

DISPLAY DEVICE 10

IMAGE ACQUISITION DEVICE 20

FIRST LIGHT SOURCE 21A

SECOND LIGHT SOURCE 21B

CAMERA 22

OUTPUT DEVICE 40

INPUT DEVICE 50

CAMERA-SUBJECT DISTANCE CALCULATOR 70

# FIG.3

# FIG.4

# FIG.5

# FIG.6

$$\begin{cases} \theta 1 = \dfrac{1}{2} \cdot arctan(a/x) \\[2mm] \theta 2 = \dfrac{1}{2} \cdot arctan(b/x) \\[2mm] d = r \cdot sin\theta 1 + r \cdot sin\theta 2 \end{cases}$$

# FIG.7

# FIG.8

# FIG.9

```
                        ┌───────────┐
                        │   START   │
                        └───────────┘
                              │
                              ▼
        ┌─────────────────────────────────────────┐
        │     TURN ON ONE OF LIGHT SOURCES        │ ⌐S101
        └─────────────────────────────────────────┘
                              │
                              ▼
        ┌─────────────────────────────────────────┐
        │   CAPTURE IMAGE OF EYEBALL BY CAMERA    │ ⌐S102
        └─────────────────────────────────────────┘
                              │
                              ▼
        ┌─────────────────────────────────────────┐
        │      TURN ON THE OTHER LIGHT SOURCE     │ ⌐S103
        └─────────────────────────────────────────┘
                              │
                              ▼
        ┌─────────────────────────────────────────┐
        │   CAPTURE IMAGE OF EYEBALL BY CAMERA    │ ⌐S104
        └─────────────────────────────────────────┘
                              │
                              ▼
        ┌─────────────────────────────────────────┐
        │       CALCULATE POSITIONS OF CORNEA     │ ⌐S105
        │        REFLECTION CENTERS ON IMAGE      │
        └─────────────────────────────────────────┘
                              │
                              ▼
        ┌─────────────────────────────────────────┐
        │     CALCULATE CENTER-CENTER DISTANCE    │ ⌐S106
        └─────────────────────────────────────────┘
                              │
                              ▼
        ┌─────────────────────────────────────────┐
        │   CALCULATE CORNEA CURVATURE RADIUS     │ ⌐S107
        └─────────────────────────────────────────┘
                              │
                              ▼
                        ┌───────────┐
                        │    END    │
                        └───────────┘
```

# FIG.10

START

TURN ON ONE OF LIGHT SOURCES — S201

CAPTURE IMAGE OF EYEBALL BY CAMERA — S202

TURN ON THE OTHER LIGHT SOURCE — S203

CAPTURE IMAGE OF EYEBALL BY CAMERA — S204

CALCULATE SETS OF COORDINATES OF PUPIL CENTER AND CORNEA REFLECTION POINT ON IMAGE — S205

CONVERT PUPIL CENTER AND CORNEA REFLECTION POINT INTO WORLD COORDINATES — S206

CALCULATE STRAIGHT LINE CONNECTING VIRTUAL LIGHT SOURCE POSITION AND CORNEA REFLECTION POINT — S207

CALCULATE CORNEA CURVATURE CENTER FROM CALCULATED STRAIGHT LINE AND CALCULATED CORNEA CURVATURE RADIUS — S208

CALCULATE LINE-OF-SIGHT VECTOR CONNECTING PUPIL CENTER AND CORNEA CURVATURE CENTER — S209

CALCULATE WORLD COORDINATES OF INTERSECTION BETWEEN LINE-OF-SIGHT VECTOR AND DISPLAY UNIT — S210

CONVERT WORLD COORDINATES INTO COORDINATES OF DISPLAY UNIT AND ACQUIRE POSITION OF POINT OF VIEW — S211

END

EP 4 183 322 A1

# FIG.11

# FIG.12

# FIG.13

23

# FIG.14

EP 4 183 322 A1

**INTERNATIONAL SEARCH REPORT**

International application No.

PCT/JP2021/008894

A. CLASSIFICATION OF SUBJECT MATTER
Int. Cl. A61B3/113(2006.01)i, G06F3/0346(2013.01)i, G06F3/038(2013.01)i
FI: G06F3/038 310A, G06F3/0346 423, A61B3/113

According to International Patent Classification (IPC) or to both national classification and IPC

B. FIELDS SEARCHED

Minimum documentation searched (classification system followed by classification symbols)
Int. Cl. A61B3/113, G06F3/0346, G06F3/038

Documentation searched other than minimum documentation to the extent that such documents are included in the fields searched
Published examined utility model applications of Japan    1922-1996
Published unexamined utility model applications of Japan  1971-2021
Registered utility model specifications of Japan          1996-2021
Published registered utility model applications of Japan  1994-2021

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2007-136000 A (NIPPON TELEGRAPH AND TELEPHONE CORP.) 07 June 2007 (2007-06-07), paragraphs [0028], [0053] | 1-5 |
| A | JP 2012-187178 A (FUJITSU LTD.) 04 October 2012 (2012-10-04), paragraph [0047] | 1-5 |

☐ Further documents are listed in the continuation of Box C.    ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
24.03.2021

Date of mailing of the international search report
18.05.2021

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

25

INTERNATIONAL SEARCH REPORT

Information on patent family members

International application No.

PCT/JP2021/008894

| Patent Documents referred to in the Report | Publication Date | Patent Family | Publication Date |
|---|---|---|---|
| JP 2007-136000 A | 07.06.2007 | (Family: none) | |
| JP 2012-187178 A | 04.10.2012 | (Family: none) | |

Form PCT/ISA/210 (patent family annex) (January 2015)

**EP 4 183 322 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020038734 A